# EUROPEAN PATENT SPECIFICATION

(11) **EP 3 417 475 B1**
(45) Date of publication and mention of the grant of the patent: **19.04.2023**
(21) Application number: 17753763.6
(22) Date of filing: 15.02.2017
(51) Int. Cl.: H01L 21/027, H01L 21/302, H01L 21/268, H01L 21/306, C12M 3/06, C12M 1/00

(54) **METHOD OF SELECTIVELY VARYING THE WETTING CHARACTERISTICS OF A SURFACE**
VERFAHREN ZUR SELEKTIVEN VERÄNDERUNG DER BENETZUNGSEIGENSCHAFTEN EINER OBERFLÄCHE
PROCÉDÉ POUR FAIRE VARIER SÉLECTIVEMENT LES CARACTÉRISTIQUES DE MOUILLAGE D'UNE SURFACE

(30) Priority: 15.02.2016 US 201662295475 P; 15.11.2016 US 201662422572 P
(43) Date of publication of application: 26.12.2018
(73) Proprietor: Newport Corporation, Irvine, CA 92606 (US)
(72) Inventor: DOMKE, Matthias, 6850 Dornbirn (AT); KOSTAL, Elisabeth, 6971 Hard (AT); STROJ, Sandra, 6971 Hard (AT); KASEMANN, Stephan, 6800 Feldkirch (AT); HENDRICKS, Frank, 6822 Satteins (AT); MATYLITSKY, Victor, 88131 Lindau (DE)
(74) Representative: Forresters IP LLP
(86) International application number: PCT/US2017/017990
(87) International publication number: WO 2017/142958

(56) References cited:
- WO-A1-2015/025093
- WO-A2-2007/096476
- US-A1- 2006 008 627
- US-A1- 2006 024 504
- US-A1- 2007 259 474
- US-A1- 2010 089 529
- US-A1- 2014 014 401
- CORE: "X-LASE Micromachining with macrospeed", INTERNET CITATION, 2005, XP007902847, Retrieved from the Internet: URL:http://www.corelase.fi/fileadmin/user_ upload/Products/X_lase_24012006.pdf [retrieved on 2007-08-29]
- ILKER S BAYER ET AL: "Modification of wetting properties of laser-textured surfaces by depositing triboelectrically charged Teflon particles", COLLOID AND POLYMER SCIENCE, SPRINGER, BERLIN, DE, vol. 291, no. 2, 18 August 2012 (2012-08-18), pages 367-373, XP035166471, ISSN: 1435-1536, DOI: 10.1007/S00396-012-2757-0

## Description

### Cross Reference to Related Applications

The present application claims priority to United States Provisional Pat. Appl. Ser. No. 62/295,475, entitled "Method of Selectively Varying the Wetting Characteristics of a Surface", filed on February 15, 2016, and United States Provisional Pat. Appl. Ser. No. 62/523,435, entitled "Method of Fabricating Fog-Collecting Surfaces and Related Devices", filed on November 15, 2016.

### Background

Wetting may be defined as a liquid's tendency to bead or sheet when in contact with a solid surface. Generally, wetting is a function of the properties of both the surface and the liquid. The adhesive forces between the liquid and the surface causes the liquid drop to spread across the surface. In contrast, the cohesive forces within the liquid drop and the chemistry/geometry of the surface may cause the liquid drop to minimize contact with the surface.

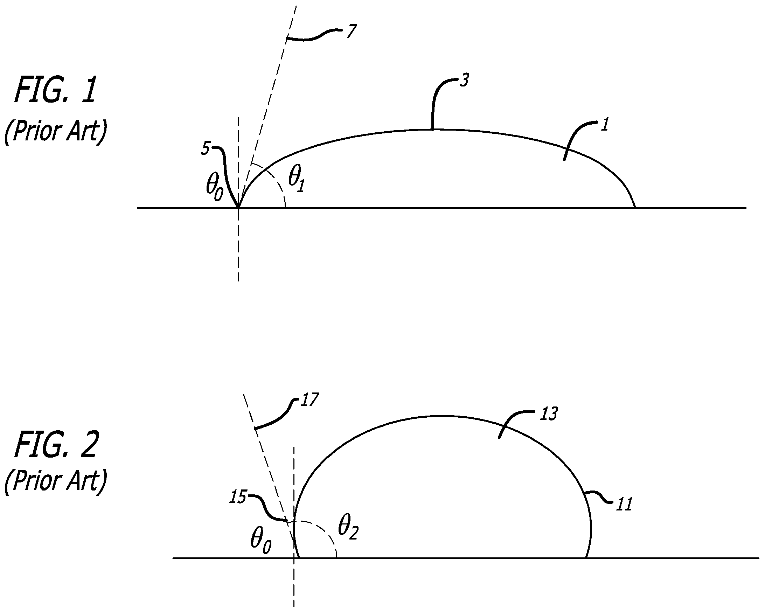

Figure 1 shows a side view of a droplet of liquid disposed on a flat surface. As shown in Figure 1, the droplet 1 includes a droplet wall 3 which is in contact with the flat or substantially flat surface 5. The contact angle 7 (Θₗ) of the droplet wall 7 and the flat surface (Θₒ) is less than ninety degrees (<90°). When the contact angle 7 (Θₗ) is less than ninety degrees (<90°) wetting or sheeting is favorable. Surfaces favorable to wetting are commonly referred to as hydrophilic surfaces.

In contrast, Figure 2 shows a side view of another droplet of liquid disposed on a flat surface. Like the droplet shown in Figure 1, the droplet 11 shown in Figure 2 includes a droplet wall 13 which is in contact with the flat or substantially flat surface 15. The contact angle 17 (Θ₂) of the droplet wall 17 and the flat surface (Θₒ) is greater than ninety degrees (>90°). When the contact angle 7 (Θ₂) is greater than ninety degrees (>90°) wetting or sheeting is unfavorable. Surfaces unfavorable to wetting are commonly referred to as hydrophobic surfaces.

Presently there is considerable research being conducted into the interactions of liquids to solid surfaces and the applications thereof. It is generally known that the surface roughness has a considerable effect of the hydrophobicity of a surface. More specifically, the enhanced surface roughness may result in the formation of microscales, nano-projections, nano-cavities which enhance the hydrophobicity of a surface. As such, research into the effects of surface roughness on fluid dynamics with regard to the formation of ultrahydrophobic surfaces (i.e. wherein droplets have a contact angle of greater than about 150°) has been increasing. In addition, other industrial applications require the development of ultrahydrophilic coatings and materials. While this research has proven informative, a number of shortcomings associated with most prior art methods of enhancing the hydrophilicity or hydrophobicity of a surface have been identified. For example, many prior art methods of enhancing the hydrophilicity or hydrophobicity of a surface require the deposition of delicate polymer and/or chemical coatings of a substrate. In addition, often, these applied coating are configured to enhance either the hydrophilicity or hydrophobicity of the surface. Moreover, generally, the hydrophilicity or hydrophobicity of the entire area of the surface was enhanced. Thus, precise selective tailoring of the hydrophilicity and/or hydrophobicity of various portions or sections of a surface has proven problematic.

Thus, in light of the foregoing, there is an ongoing need for method of selectively varying the wetting characteristics of a surface.
US 2006/008627 A1 discloses a method of plating a substrate including coating a substrate surface, laser-treating a region of the coated surface, and plating the region.
ILKER S BAYER ET AL: "Modification of wetting properties of laser-textured surfaces by depositing triboelectrically charged Teflon particles", COLLOID AND POLYMER SCIENCE, vol. 291, no. 2, 18 August 2012, pages 367-373, XP03516647 discloses hydrophilic laser-textured silicon wafers with natural oxide surfaces rendered hydrophilic by depositing electrostatically charged submicrometer Teflon particles, a process termed as triboelectric Teflon adhesion.

### Summary

The present application is directed to various methods of selectively varying the wetting characteristics of the surface. In one embodiment, the present application is directed to a method for selectively enhancing the wetting characteristics of a substrate and includes providing at least one substrate having a first wetting characteristic. Thereafter, at least one laser system is configured to output at least one patterning beam to the substrate. The patterning beam has a wavelength of 200nm to 1200nm and laser energy greater than the ablation threshold of the substrate. One or more features are selectively formed on at least a portion of the surface of the substrate with the patterning beam. The feature formed by the patterning beam has a second wetting characteristic wherein the first wetting characteristic and second wetting characteristic are different. Thereafter, at least one substrate coating is selectively applied to the substrate and the feature. Finally, at least a portion of the substrate coating is selectively ablated from the substrate to expose the feature formed on the substrate.

In addition, the present application discloses a method for selectively enhancing the wetting characteristics of a substrate and includes the steps of providing at least one Silicon substrate having a first wetting characteristic. In addition, at least one laser system configured to output at least one patterning beam having a wavelength 500nm to 550nm is provided. The patterning beam, which has laser energy greater than the ablation threshold of the substrate, is directed onto the substrate. At least one feature is selectively formed on at least a portion of the surface of the substrate with the patterning beam. The area of the substrate having feature formed thereon has a second wetting characteristic wherein the first wetting characteristic and second wetting characteristic are different. At least one hydrophobic fluorocarbon coating substrate coating is applied to the substrate and the feature. Thereafter, at least a portion of the hydrophobic fluorocarbon coating substrate is selectively ablated from the portion of the substrate to expose the feature.

Further, the present application discloses another method for selectively enhancing the wetting characteristics of a substrate and includes the steps of providing at least one Silica or borosilicate substrate having a first wetting characteristic. At least one laser system configured to output at least one patterning beam to the substrate is provided. The patterning beam has laser energy greater than the ablation threshold of the substrate. Interconnected channels are formed on at least a portion of the surface of the substrate with the patterning beam, the interconnected channels having a second wetting characteristic wherein the first wetting characteristic and second wetting characteristic are different. At least one hydrophobic fluorocarbon coating substrate coating is selectively applied to the substrate and the interlocking channels. Thereafter,
at least a portion of the hydrophobic fluorocarbon coating substrate is selectively ablated from a portion of the substrate to expose the interlocking channels.

Other features and advantages of the methods for selectively varying the wetting characteristics of a surface as described herein will become more apparent from consideration of the following detailed description.

### Brief Description of the Drawings

Various embodiments of the methods for varying the wetting characteristics of a surface will be will be explained in more detail by way of the company drawings, wherein:
Figure 1 shows a planar side view of a drop of liquid positioned on a hydrophilic surface;
Figure 2 shows a planar side view of a drop of liquid positioned on a hydrophobic surface;
Figure 3 shows a flowchart of a method of selectively varying the wetting characteristics of a surface;
Figure 4 shows a cross-sectional view of a substrate having a patterning beam creating one or more features on a substrate in accordance the methods of selectively varying the wetting characteristics of a substrate disclosed herein;
Figure 5 shows a cross-sectional view of a substrate having a surface coating applied to one or more features formed on a substrate in accordance the methods of selectively varying the wetting characteristics of a substrate disclosed herein;
Figure 6 shows a cross-sectional view of a ablative laser signal selectively ablating a surface coating applied to one or more features formed on a substrate in accordance the methods of selectively varying the wetting characteristics of a substrate disclosed herein;
Figure 7 shows a cross-sectional view of an embodiment of a cell growth system relying on the microfluidic processing capabilities of a device manufactured using the methods of selectively varying the wetting characteristics of a substrate;
Figure 8 shows an elevated perspective view of an embodiment of a single cell singulation system manufactured using the methods of selectively varying the wetting characteristics of a substrate;
figure 9 shows a photograph of him their locking channels formed on the substrate having been manufactured in accordance with the methods for selectively varying the wetting characteristics of a substrate as disclosed herein;
Figure 10 shows an elevated planar view of ablation regions formed on a substrate having been manufactured in accordance with the methods for selectively varying the wetting characteristics of a substrate as disclosed herein;
figure 11 shows a schematic diagram of an embodiment of a fog collection system which incorporates a substrate manufactured in accordance with the methods for selectively varying the wetting characteristics of a substrate as disclosed herein;
Figure 12 shows graphically the performance characteristics of a substrate having been laser structured, passivated, and patterned in accordance with the methods for selectively varying the wetting characteristics of the substrate as disclosed herein; and
Figure 13 shows another graph presenting the performance characteristics of a substrate having been laser structured, passivated, and patterned in accordance with the methods for selectively bearing the wetting characteristics of the substrate as disclosed herein.

### Detailed Description

The present application is directed to various methods for selectively varying the wetting characteristics of a surface and applications thereof. More specifically, the present application discloses methods for selectively enhancing the hydrophobicity of at least a portion of a substrate. In addition, the present application discloses methods for selectively enhancing the hydrophilicity of at least a portion of a substrate. Further, the present application discloses methods for selectively enhancing the hydrophobicity of a substrate while also selectively enhancing the hydrophilicity of another portion of the same substrate. In another embodiment, the present application discloses various methods of fabricating fog-collecting surfaces, atmospheric water generators, and similar devices configured to collect or harvest water or other fluids from an atmosphere (hereinafter "fog-collecting")

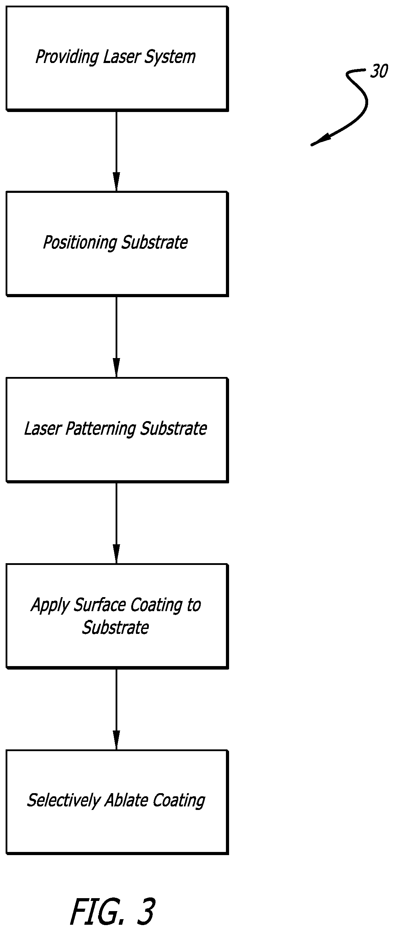

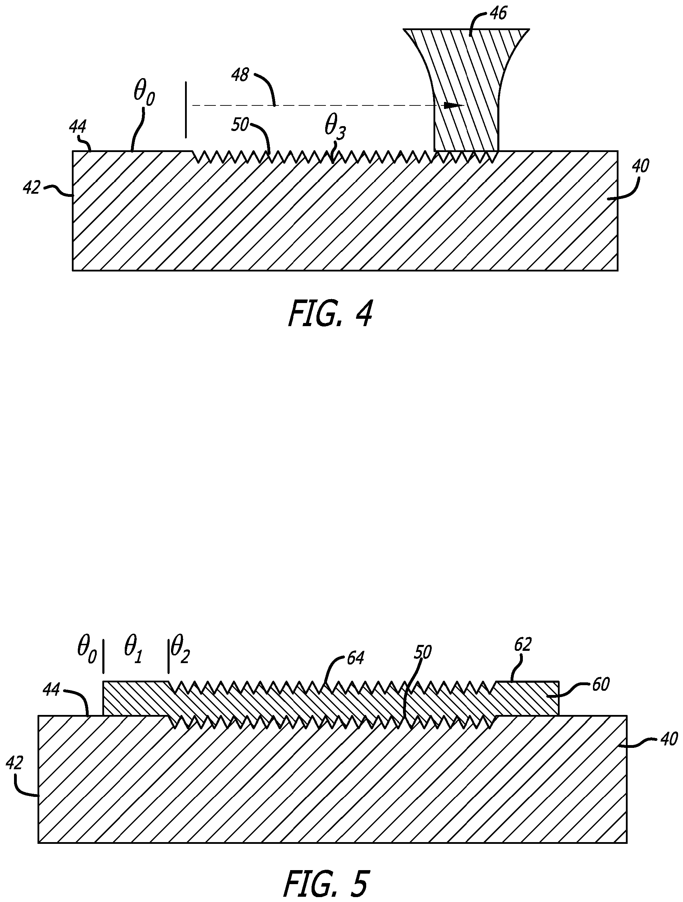

Figure 3 shows a flow chart of a method for selectively enhancing varying the wetting characteristics of a substrate, while Figures 4-6 shows a more detailed view of each step of the process. As shown, the method for selectively varying the wetting characteristics of a substrate 30 includes providing at least one laser patterning system or similar pattern device (hereinafter laser system). In one embodiment, the laser system comprises at least one laser-based texturing system configured to output at least one patterning beam 46 incident on the substrate 40 in optical communication therewith. In one embodiment, the laser system comprises a Spirit Laser System^{™}, manufactured by Spectra-Physics, Inc. For example, in one embodiment, the laser system comprises a laser system configured to output a patterning beam 46 having a wavelength of about 400 nm to about 1100 nm. In another embodiment, laser system is configured to output a patterning beam 46 having a wavelength of about 400 nm to about 600 nm. In yet another embodiment, the patterning beam 46 has a wavelength of about 520 nm. Optionally, the laser system may be configured to output multiple patterning beams 46 configured to form multiple patterns of the substrate 40 simultaneously or, in the alternative, sequentially on the substrate 40.

Referring again to Figures 3-6, the laser system may be configured to output at least one patterning beam 46 may have a frequency from single shot operation to ~1kHz (e.g. output from Ti:sapphire laser system) of about 10 kHz to about 1000 kHz. For example, in one embodiment, the patterning beam 46 has a frequency of about 200 kHz, although those skilled in the art will appreciate that the patterning beam 46 may have any desired frequency. Further, the patterning beam 46 may have a spot radius from about .1 µm to about 100 µm. In another embodiment, the patterning beam 46 has a spot radius of about 1 µm to about 10 µm. In a more specific embodiment the patterning beam 46 has a spot radius of about 6 µm, although those skilled in the art will appreciate that the laser system may be configured to output at least one patterning beam 46 having any desired spot radius. In one embodiment, the pulse energy of the patterning beam 46 output by the laser system is about 1µJ to 25µJ. For example, in a more specific embodiment, the energy of the pattern beam 46 is about 7.3 µJ. For those skilled in the art the pulse energy could be scaled to any value.

Optionally, the laser system may be configured to output at least one patterning beam having any fluence from about 1 J/cm² to about 40 J/cm². For example, in one embodiment the laser system is configured to output a patterning beam 46 having a fluence of about 10 J/cm² to about 15 J/cm². In a more specific embodiment, the patterning beam 46 has a fluence of about 12.9 J/cm². Optionally, any fluence above the ablation/modification threshold of the substrate material may be used. In one embodiment, the patterning beam 46 is configured to be scanned or otherwise transposed across the substrate 40. For example, in one embodiment, the scan speed of the patterning beam 46 may be about 1 mm/s to about 5 m/s or more. Optionally, a first patterning beam 46 may be scanned across the substrate 40 at a rate of about 240 mm/s while at least a second patterning beam 46 is scanned across the substrate 40 at a rate of about 1 m/s. In addition, the patterning beam 46 may have a pulse duration from about 100 fs to about 10 µs. In one embodiment, the patterning beam 46 has a pulse duration of about 5 ns to about 400 ns. Optionally, the patterning beam 46 may have a pulse duration of about 5 ns to about 100 ns. Further, multiple patterning beams 46, each configured to form one or more patterns of the same or differing size, depth, spacing, transverse dimension, fluence, energy, wavelength, frequency, and the like may be simultaneously or sequentially scanned across at least a portion of the substrate 40.

Referring again to Figures 3-6, the substrate 40 may comprise Silicon. In another embodiment, the substrate 40 comprises Germanium. Optionally, any variety of materials may be used to form the substrate 40, including, without limitations, various alloys, metals, brass, stainless steel, copper, aluminum, titanium, glass, polymers, elastomers, composite materials, fabrics, mammalian tissue, animal tissue, cellular material, plant tissue, fibers, and the like. As shown in Figure 4, the substrate 40 includes a substrate body 42 having at least one surface 44 configured to have at least one pattern and/or texture applied thereto or formed thereon. In one embodiment, the surface 44 of the substrate body 42 has a contact angle Θ₀, which represents the hydrophilicity and/or hydrophobicity of the substrate 40. As shown, movement 48 of at least one patterning beam 46 across the surface 44 of the substrate body 42 results in the formation of at least one patterned area, surface discontinuity, and/or feature 50 (hereinafter features 50) having at least one contact angle Θ₃ formed in the surface 44. Those skilled in the art will appreciate that any number, variety, shape, depth, line spacing, orientation, and/or frequency of features 50 having any desired smoothness or roughness may be formed on the surface 44. For example, in the illustrated embodiment the features 50 comprise a generally saw-tooth profile, although those skilled in the art will appreciate that the features 50 may be formed in any variety of shapes and/or profiles.

As shown in Figures 3-5, at least one surface coating may be applied to at least a portion of the surface 44 of the substrate body 42 following the formation of one or more patterned areas and/or features 50 on the surface 44 of the substrate 40. As shown in Figure 5, a surface coating 60 may be deposited the surface 44 distally from the patterned area and proximate to the feature 50. In the illustrate embodiment the coating layer 60 includes at least one featureless region 62 having a contact angle Θ₁ and at least one patterned region 64 having a contact angle Θ₂ wherein the pattern region 64 approximates the features 50 formed on the surface 44. Those skilled in the art will appreciate that any variety surface coatings 60 may be applied. For example, in one embodiment the thin film coating comprising one or more fluoropolymers, Teflon-like materials, and the like. In one embodiment, one or more plasma-polymerized fluorocarbon films may be used to form an ultra-hydrophobic layer 60 on the features 50 formed on the surface 44. Optionally, the surface coating 60 may comprise C₄F₈ or similar materials. Alternate coating materials include, without limitations, Teflon, Sol-Gels, Silicon Dioxide (SiO₂), hydrophilic polymers, hydrophobic polymers, fluorosilan, laser-induced hydrophilic coatings, laser-induced hydrophobic coatings, and the like. In another embodiment, one or more laser - induced nanostructures may be formed as the coating layer 60. Those skilled in the art will appreciate that the coating layer 60 may be applied to the surface 44 using any variety of coating processes known in the art, including, without limitations, vapor-deposition and the like.

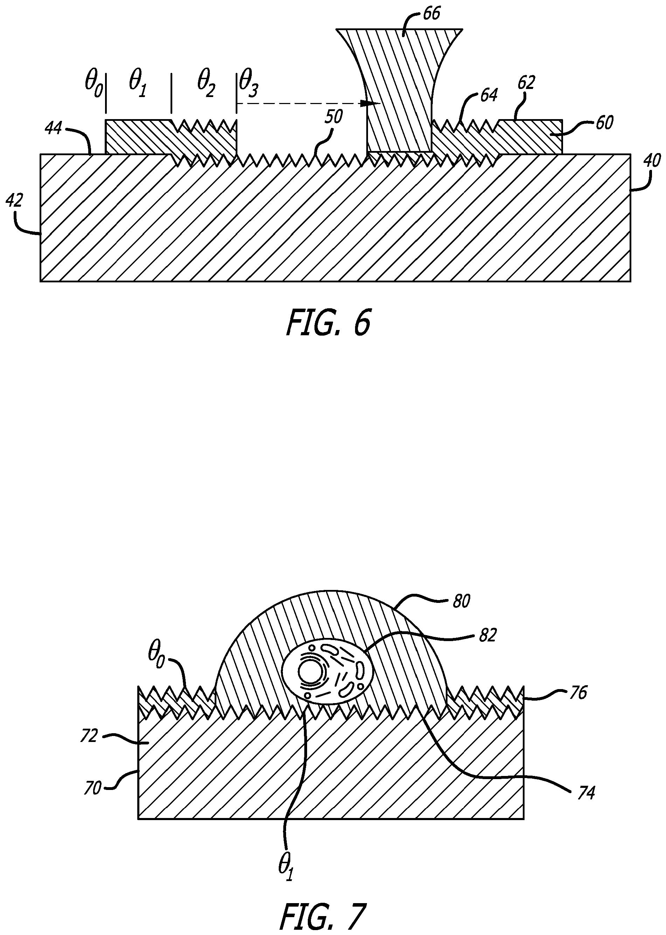

Referring again to Figures 3-6, at least a portion of the coating layer 60 may be selectively ablated or otherwise removed from the surface 44 of the substrate body 42. As shown in Figure 6, in one embodiment at least one ablative laser signal 66 may be used to selectively ablate at least a portion of the coating layer 66. In the illustrated embodiment, at least a portion of the coated pattern region 64, having a contact angle of Θ₂ is selectively ablated to reveal the features 50 formed on the surface 44 of the substrate 40 having a contact angle of Θ₃. In one embodiment, the ablative laser signal 66 is generated by the laser device 32 used to form the features 50 in the substrate 40. In another embodiment, an ablative laser signal 66 is generated by an addition laser system. Optionally, at least a portion of the coated pattern region 64 may be ablated using an inductive coupled plasma deep reactive ion etching process, chemical etching, mechanical etching processes, and the like. As shown in Figure 6, a substrate 40 having multiple regions of differing hydrophilicity and/or hydrophobicity (e.g. contact angles Θ₀, Θ₁, Θ₂, Θ₃, . . . Θₙ₎ selectively formed thereon may be easily and quickly manufactured. In addition, those skilled in the art will appreciate that the any number, size, orientations, and/or hydrophilic/hydrophobic characteristics may be selectively applied to any variety of substrates.

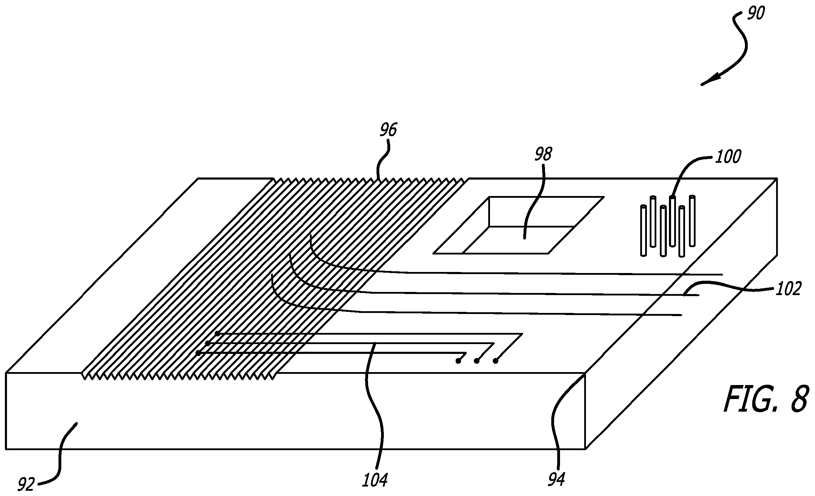

The ability to selective vary or alter the wetting characteristics of a surface has a wide variety of applications, For example, Figure 7 shows an embodiment of a cell-growth system relying on the microfluidic processing capabilities of device manufactured using the aforementioned methods. More specifically, the substrate 70 comprising a substrate body 72 having at least one patterned region 74 having a contact angle of Θ₁ may be manufactured. One or more wetting-enhancing coatings 76 having a contact angle of Θ₀ may be applied to the substrate body 72. As described above, the wetting characteristics of the patterned area 74 and the coated area 76 differ. Thereafter, at least one culture 82 present within a culture medium 80 may be selectively applied to at least one of the patterned region 74 and/or the coated region 76. As shown in Figure 8, single cell singulation systems and devices for single cell singulation may be manufactured using the methods described herein. For example, a multiple feature singulation system 90 may be constructed by directing one or more laser beams 94 onto a substrate 92. The laser beams 94 may be configured to form various features on the substrate 92. In the illustrated embodiment, the substrate 92 includes function areas 96 having the wetting characteristics selectively altered as described above. These functional areas 96 may be in communication with one or more cavities 98, passages or through holes 100, waveguides 102, conducting areas, and the like formed on at least one surface of the substrate 92.

In another embodiment, the method described herein may be used to fabricate fog-collecting surfaces, atmospheric water generators, and similar devices configured to collect or harvest water or other fluids from an atmosphere (hereinafter "fog-collecting"). More specifically, the present application discloses methods for selectively modifying the hydrophobicity and/or hydrophilicity of at least a portion of a substrate to enhancing the fog-collecting efficiency of at least one surface of the substrate using the process described in Figures 3-6. In another embodiment, the methods described herein may be used to enhance antifogging surfaces, anti-icing surfaces, and/or oleophobic surfaces.

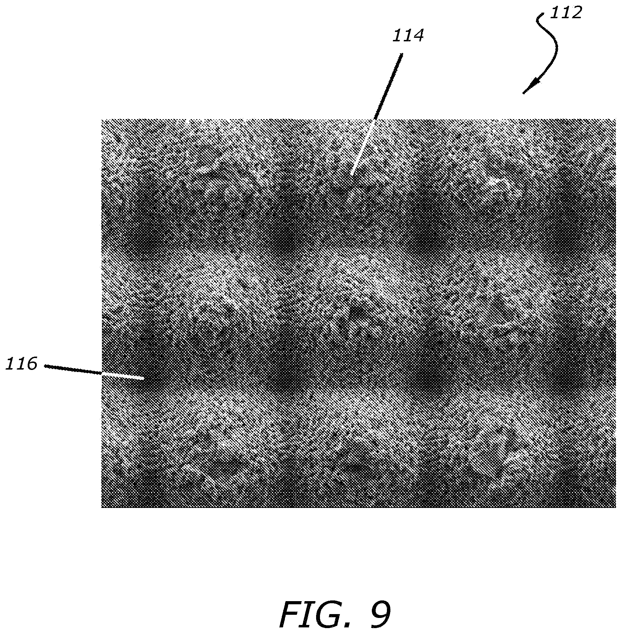

As shown in Figures 3-6, 9, and 10, in one embodiment, the patterning beam 46 is configured to be scanned or otherwise transposed across the substrate 40. For example, in one embodiment, the scan speed of the patterning beam 46 may be about 1 mm/s to about 10 m/s or more. For example, in one embodiment, the scan speed of the patterning beam 46 is about 1 m/s. Further, the patterning beam 46, the laser system (not shown), and/or the substrate 40 may be moved along any desired axis to achieve scanning the patterning beam across at least a portion of the substrate 40. For example, in one embodiment, the laser system (not shown) is moved laterally (X-axis), longitudinally (Y-Axis), and/or raised and lowered (Z-axis) relative to the location of the substrate 40. Similarly, the substrate 40 may be moved laterally (X-axis), longitudinally (Y-Axis), and/or raised and lowered (Z-axis) relative to the location of the laser system (not shown). Further, the patterning beam 46may be orthogonal to the surface of the substrate. In another embodiment, the patterning beam 46 need not be orthogonal to the surface of the substrate 40. Optionally, one or more beam steering systems, mirrors, fiber optic devices, waveguides, and the like may be used to permit rapid and precise scanning the patterning beam across at least a portion of the substrate 40. Further, the laser system may be configured to output multiple patterning beams 46 for laser patterning the substrate simultaneously and/or sequentially.

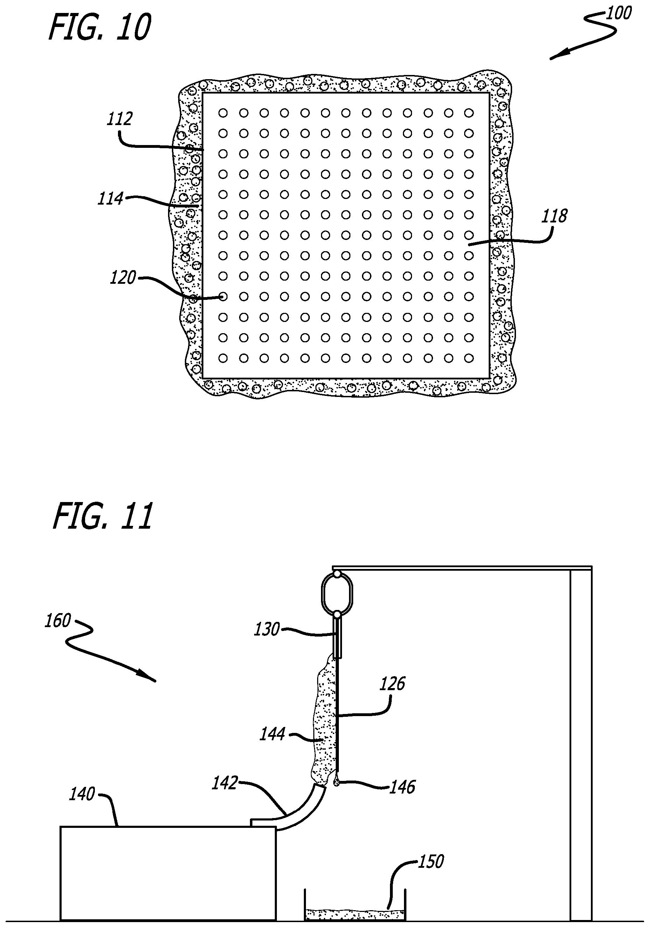

Referring again to Figures 3-6, 9, and 10 the substrate 40 may be laser patterned by irradiating at least a portion of the substrate with the patterning beam 46 from the laser system. In one embodiment, at least one surface of the substrate 40 has at least one laser-generated pattern formed thereon. For example, as shown in Figure 10, a series of interconnected channels or features 116 may be formed on a surface 114 of the substrate 112 by the laser system described above as imaged by a scanning electron microscope. As shown, the formation of the various patterns 116 formed on the substrate 112 may result in the surface 114 of the substrate 112 becoming more hydrophilic as compared with a non-patterned substrate. In another embodiment, the substrate 112 may have numerous channels, ridges, shapes, features, patterns and the like formed thereon. In another embodiment, the surface 114 may have multiple patterns formed on various regions of the substrate 112.

Referring again to Figures 3-6, 9, and 10, one or more substrate coatings 118 may be applied to at least a portion of the surface 114 of the substrate112. For example, in one embodiment, at least one layer of a hydrophobic fluorocarbon coating was applied to the patterned surface 114 of the substrate 112. Exemplary fluorocarbons include, without limitations, CF₂, CF₃, various polymers such as polypropylene, polydimethylsiloxane, Teflon, Carbon nano-tubes, graphene, fluorosilane and similar materials. Optionally, one or more nanostructures may be formed on at least a portion of the substrate 114, the nanostructures configured to selectively enhance hydrophilicity and/or hydrophobicity. Those skilled in the art will appreciate that any variety of materials may be used to form at least one hydrophobic layer on the substrate 112. In another embodiment, one or more hydrophilic layers may be deposited on the patterned substrate, including, for example, TiO₂ and similar materials.

As shown in Figures 3-6, 9, and 10, the coated patterned substrate 112 may then be further processed to include one or more ablation regions 120 on the surface 114 of the substrate 112. For example, in one embodiment, at least one ablation region 120 comprises an area wherein substantially all the substrate coating 118 is selectively removed from the surface 114 of the substrate 112, thereby exposing the underlying patterns 116 formed on the surface 114. In one embodiment, the laser system may be used to selective ablate the substrate coating 118 from the surface 114. Optionally, any variety of devices and methods may be used to selectively ablate the substrate coating 118, including, without limitations, laser ablation, chemical ablation, mechanical ablation, and the like. As such, the surface 114 consists of one or more hydrophilic patterns 116 accessible through the ablation regions 120 formed in the substrate coating 118. Figure 10 shows a processed substrate device 120 wherein the substrate 12 includes multiple ablation regions 120 formed in the substrate coating 118 applied to the surface 114.

### Experimental Results

Various substrates where prepared to examine the fog-collecting efficiency of devices manufactured using the laser structured+passivated+patterned process described in in the present application as compared to devices manufactured using alternate methods. In specific embodiment, the fabrication process of the laser structured+passivated+patterned sample was as follows:
1. Structuring of a Pyrex wafer: the wavelength was 520 nm, the focus radius was 6 µm, the fluence was varied between 3.8 J/cm² (ablation threshold) and 12.6 J/cm², the scan speed was set to 1 m/s, the, line distance of the hatch pattern was between 10 µm and 50 µm (the optimal case is when the line distance is equal or slightly larger than the spot diameter), the laser was scanned 20 times across the sample.
2. A Teflon-like polymer (CF₂) was deposited by a plasma process to make the surface superhydrophobic.
3. Circular areas (500 µm diameter, 1 mm distance) of the Teflon-like polymer coating were ablated. The hatch line distance was set to 2.5 µm, the scan speed was set to 500 mm/s, the fluence to 2.1 J/cm2, the surface was scanned by single pass.

Six (6) samples were prepared, each sample having a unique architecture. The prepared sample were as follows:
**1.** Blank - unprocessed substrate (-)
**2.** Blank+passivated - coated substrate (Step 2)
**3.** Blank+passivated+patterned - coated substrate with ablation regions therein (Step 2+3)
**4.** Laser-structured - laser patterned uncoated substrate (Step 1)
**5.** Laser-structured+passivated - laser patterned coated substrate (Step 1+2)
**6.** Laser-structured +passivated+patterned - laser patterned coated substrate with ablation regions (Step 1+2+3)

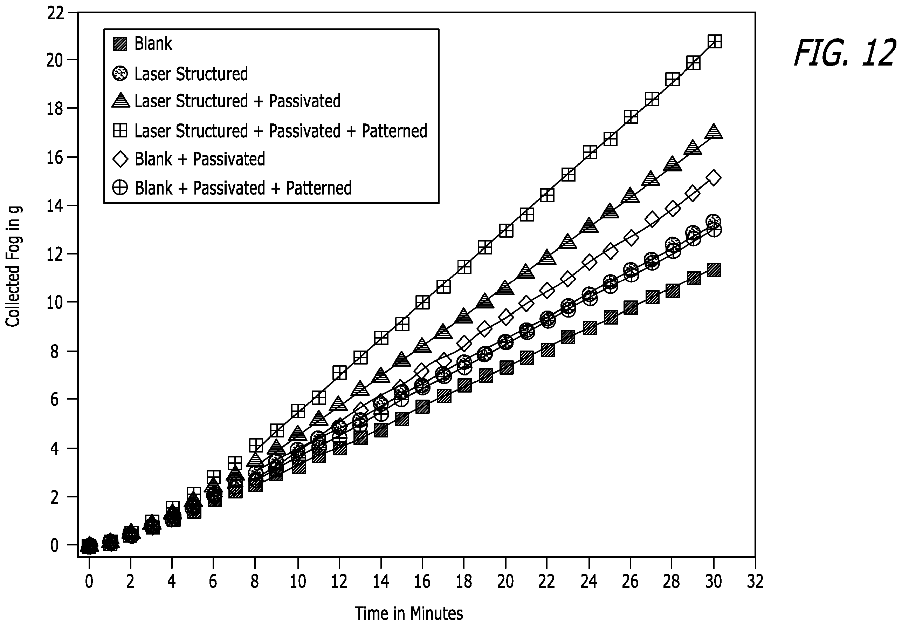

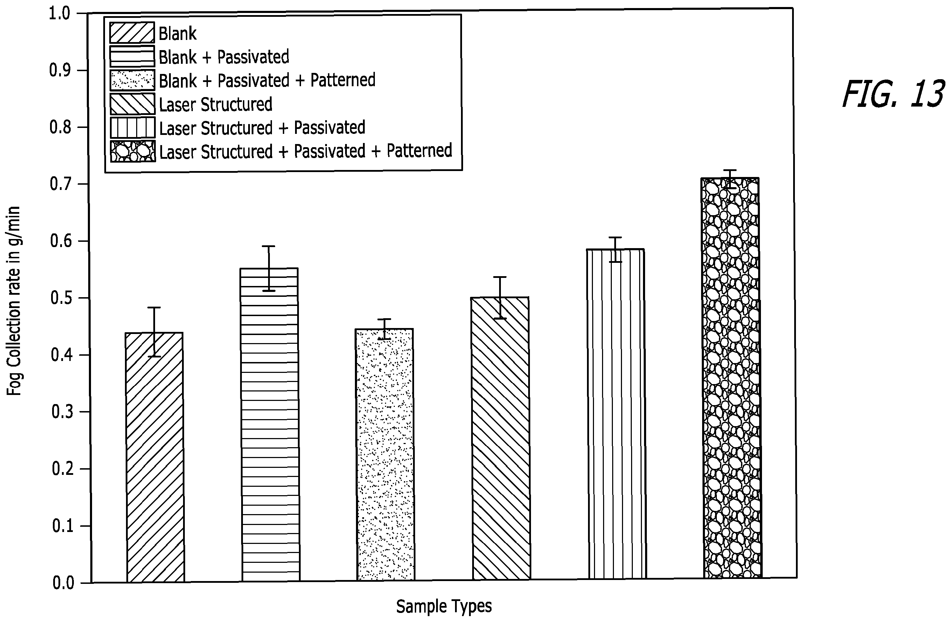

A test was prepared to compare the fog-collecting performance of each sample described above under substantially identical conditions. Figure 11 shows an embodiment of the test system 160 wherein each test sample 126 was supported by a substrate support 130 positioned proximate to a nebulizer 140. As shown, each test sample 126 was arranged in a vertical orientation proximate to a nebulizer outlet 142 which directed water vapor 144 to the test samples 126 for a pre-determined duration. A containment vessel 150 was positioned below the test sample 126 to collect water collected by and falling 146 from the test sample 126. The volume of water in the containment vessel 150 was weighted or otherwise examined to determine the fog-collecting efficiency of each test sample 126. As shown in Figures 12 and 13, the sample manufactured using the laser-structured +passivated+patterned methods described in the present application produced the highest efficiency fog-collecting devices.

The embodiments disclosed herein are illustrative of the principles of the invention. Other modifications may be employed which are within the scope of the invention. Accordingly, the devices disclosed in the present application are not limited to that precisely as shown and described herein.

## Claims

1. A method for selectively enhancing the wetting characteristics of a substrate, comprising:
providing at least one substrate, the substrate having a first wetting characteristic;
providing at least one laser system configured to output at least one patterning beam having a wavelength of 200nm to 1200nm to the substrate, the patterning beam having an laser energy greater than the ablation threshold of the substrate;
selectively forming at least one feature on at least a portion of the surface of the substrate with the patterning beam, the feature having a second wetting characteristic wherein the first wetting characteristic and second wetting characteristic are different;
selectively applying at least one substrate coating to the substrate and the feature; and
selectively ablating at least a portion of the substrate coating from the substrate to expose the feature formed on the substrate.

2. The method of claim 1 wherein further comprising providing a Silicon substrate having a first wetting characteristic.

3. The method of claim 1 wherein further comprising providing a Germanium substrate having a first wetting characteristic.

4. The method of claim 1 wherein the patterning beam has a wavelength of 400 nm to 1000 nm.

5. The method of claim 1 wherein the patterning beam has a wavelength of 400 nm to 600 nm.

6. The method of claim 1 wherein the patterning beam has a wavelength of 500 nm to 530 nm.

7. The method of claim 1 further comprising selectively forming at least one feature on at least a portion of the surface of the substrate with one patterning beam.

8. The method of claim 1 further comprising selectively forming at least one feature on at least a portion of the surface of the substrate with multiple patterning beams.

9. The method of claim 1 wherein the substrate coating is applied using a vapor deposition process.

10. The method of claim 1 further comprising applying a fluoropolymer to the substrate as the substrate coating.

11. The method of claim 1 further comprising applying a plasma-polymerized fluorocarbon to the substrate as the substrate coating to form an ultra-hydrophobic layer on the substrate.

12. The method of claim 1 wherein the second wetting characteristic is more hydrophobic than the first wetting characteristic.

13. The method of claim 1 wherein the second wetting characteristic is more hydrophilic than the first wetting characteristic.

14. The method of claim 1 wherein:
the substrate is a Silicon substrate;
the patterning beam has a wavelength from 500 to 550nm; and
the substrate coating is a fluoropolymer substrate coating.

15. A method for selectively enhancing the wetting characteristics of a substrate, comprising:
providing at least one borosilicate substrate, the borosilicate substrate having a first wetting characteristic;
providing at least one laser system configured to output at least one patterning beam to the borosilicate substrate, the patterning beam having an laser energy greater than the ablation threshold of the borosilicate substrate;
selectively forming interconnected channels on at least a portion of the surface of the borosilicate substrate with the patterning beam, the interconnected channels having a second wetting characteristic wherein the first wetting characteristic and second wetting characteristic are different;
selectively applying at least one hydrophobic fluorocarbon coating substrate coating to the borosilicate substrate and the interlocking channels; and
selectively ablating at least a portion of the hydrophobic fluorocarbon coating substrate from the a portion of the borosilicate substrate to expose the interlocking channels.

## Patentansprüche

1. Verfahren zum selektiven Verbessern der Benetzungseigenschaften eines Substrats, umfassend:
Bereitstellen mindestens eines Substrats, wobei das Substrat eine erste Benetzungseigenschaft aufweist;
Bereitstellen mindestens eines Lasersystems, das dazu konfiguriert ist, mindestens einen Strukturierungsstrahl mit einer Wellenlänge von 200 nm bis 1200 nm auf das Substrat auszugeben, wobei der Strukturierungsstrahl eine Laserenergie aufweist, die größer als der Ablationsschwellenwert des Substrats ist;
selektives Ausbilden mindestens eines Merkmals auf mindestens einem Abschnitt der Oberfläche des Substrats mit dem Strukturierungsstrahl, wobei das Merkmal eine zweite Benetzungseigenschaft aufweist, wobei die erste Benetzungseigenschaft und die zweite Benetzungseigenschaft unterschiedlich sind;
selektives Aufbringen mindestens einer Substratbeschichtung auf das Substrat und das Merkmal; und
selektives Ablatieren mindestens eines Abschnitts der Substratbeschichtung von dem Substrat, um das auf dem Substrat gebildete Merkmal freizulegen.

2. Verfahren nach Anspruch 1, ferner umfassend Bereitstellen eines Siliziumsubstrats, das eine erste Benetzungseigenschaft aufweist.

3. Verfahren nach Anspruch 1, ferner umfassend Bereitstellen eines Germaniumsubstrats, das eine erste Benetzungseigenschaft aufweist.

4. Verfahren nach Anspruch 1, wobei der Strukturierungsstrahl eine Wellenlänge von 400 nm bis 1000 nm aufweist.

5. Verfahren nach Anspruch 1, wobei der Strukturierungsstrahl eine Wellenlänge von 400 nm bis 600 nm aufweist.

6. Verfahren nach Anspruch 1, wobei der Strukturierungsstrahl eine Wellenlänge von 500 nm bis 530 nm aufweist.

7. Verfahren nach Anspruch 1, ferner umfassend selektives Ausbilden mindestens eines Merkmals auf mindestens einem Abschnitt der Oberfläche des Substrats mit einem Strukturierungsstrahl.

8. Verfahren nach Anspruch 1, ferner umfassend selektives Ausbilden mindestens eines Merkmals auf mindestens einem Abschnitt der Oberfläche des Substrats mit mehreren Strukturierungsstrahlen.

9. Verfahren nach Anspruch 1, wobei die Substratbeschichtung unter Verwendung eines Dampfabscheidungsprozesses aufgebracht wird.

10. Verfahren nach Anspruch 1, ferner umfassend Aufbringen eines Fluorpolymers auf das Substrat als die Substratbeschichtung.

11. Verfahren nach Anspruch 1, ferner umfassend Aufbringen eines plasmapolymerisierten Fluorkohlenstoffs auf das Substrat als die Substratbeschichtung, um eine ultra-hydrophobe Schicht auf dem Substrat auszubilden.

12. Verfahren nach Anspruch 1, wobei die zweite Benetzungseigenschaft hydrophober ist als die erste Benetzungseigenschaft.

13. Verfahren nach Anspruch 1, wobei die zweite Benetzungseigenschaft hydrophiler ist als die erste Benetzungseigenschaft.

14. Verfahren nach Anspruch 1, wobei:
das Substrat ein Siliziumsubstrat ist;
der Strukturierungsstrahl eine Wellenlänge von 500 bis 550 nm aufweist; und die Substratbeschichtung eine Fluorpolymer-Substratbeschichtung ist.

15. Verfahren zum selektiven Verbessern der Benetzungseigenschaften eines Substrats, umfassend:
Bereitstellen mindestens eines Borsilikatsubstrats, wobei das Borsilikatsubstrat eine erste Benetzungseigenschaft aufweist;
Bereitstellen mindestens eines Lasersystems, das dazu konfiguriert ist, mindestens einen Strukturierungsstrahl auf das Borsilikatsubstrat auszugeben, wobei der Strukturierungsstrahl eine Laserenergie aufweist, die größer als der Ablationsschwellenwert des Borsilikatsubstrats ist;
selektives Ausbilden miteinander verbundener Kanäle auf mindestens einem Abschnitt der Oberfläche des Borsilikatsubstrats mit dem Strukturierungsstrahl, wobei die miteinander verbundenen Kanäle eine zweite Benetzungseigenschaft aufweisen, wobei die erste Benetzungseigenschaft und die zweite Benetzungseigenschaft unterschiedlich sind;
selektives Aufbringen mindestens einer hydrophoben Fluorkohlenstoffbeschichtungs-Substratbeschichtung auf das Borsilikatsubstrat und die ineinandergreifenden Kanäle; und
selektives Ablatieren mindestens eines Abschnitts des hydrophoben Fluorkohlenstoffbeschichtungssubstrats von dem einen Abschnitt des Borsilikatsubstrats, um die ineinandergreifenden Kanäle freizulegen.

## Revendications

1. Procédé pour améliorer sélectivement les caractéristiques de mouillage d'un substrat, comprenant :
la fourniture d'au moins un substrat, le substrat ayant une première caractéristique de mouillage ;
la fourniture d'au moins un système laser conçu pour émettre au moins un faisceau de formation de motif ayant une longueur d'onde de 200 nm à 1 200 nm vers le substrat, le faisceau de formation de motif ayant une énergie laser supérieure au seuil d'ablation du substrat ;
la formation sélective d'au moins un élément sur au moins une partie de la surface du substrat à l'aide du faisceau de formation de motif, l'élément ayant une seconde caractéristique de mouillage, la première caractéristique de mouillage et la seconde caractéristique de mouillage étant différentes ;
l'application sélective d'au moins un revêtement de substrat sur le substrat et l'élément ; et
l'ablation sélective d'au moins une partie du revêtement de substrat depuis le substrat pour faire apparaître l'élément formé sur le substrat.

2. Procédé selon la revendication 1 comprenant en outre la fourniture d'un substrat de silicium ayant une première caractéristique de mouillage.

3. Procédé selon la revendication 1 comprenant en outre la fourniture d'un substrat de germanium ayant une première caractéristique de mouillage.

4. Procédé selon la revendication 1 dans lequel le faisceau de formation de motif a une longueur d'onde de 400 nm à 1 000 nm.

5. Procédé selon la revendication 1 dans lequel le faisceau de formation de motif a une longueur d'onde de 400 nm à 600 nm.

6. Procédé selon la revendication 1 dans lequel le faisceau de formation de motif a une longueur d'onde de 500 nm à 530 nm.

7. Procédé selon la revendication 1 comprenant en outre la formation sélective d'au moins un élément sur au moins une partie de la surface du substrat à l'aide d'un faisceau de formation de motif.

8. Procédé selon la revendication 1 comprenant en outre la formation sélective d'au moins un élément sur au moins une partie de la surface du substrat à l'aide de multiples faisceaux de formation de motif.

9. Procédé selon la revendication 1 dans lequel le revêtement de substrat est appliqué selon un procédé de dépôt en phase vapeur.

10. Procédé selon la revendication 1 comprenant en outre l'application d'un polymère fluoré sur le substrat en tant que revêtement de substrat.

11. Procédé selon la revendication 1 comprenant en outre l'application d'un fluorocarbure polymérisé au plasma sur le substrat en tant que revêtement de substrat pour former une couche ultra-hydrophobe sur le substrat.

12. Procédé selon la revendication 1 dans lequel la seconde caractéristique de mouillage est plus hydrophobe que la première caractéristique de mouillage.

13. Procédé selon la revendication 1 dans lequel la seconde caractéristique de mouillage est plus hydrophile que la première caractéristique de mouillage.

14. Procédé selon la revendication 1 dans lequel :
le substrat est un substrat de silicium ;
le faisceau de formation de motif a une longueur d'onde de 500 à 550 nm ;et le revêtement de substrat est un revêtement de substrat au polymère fluoré.

15. Procédé pour améliorer sélectivement les caractéristiques de mouillage d'un substrat, comprenant :
la fourniture d'au moins un substrat de borosilicate, le substrat de borosilicate ayant une première caractéristique de mouillage ;
la fourniture d'au moins un système laser conçu pour émettre au moins un faisceau de formation de motif vers le substrat de borosilicate, le faisceau de formation de motif ayant une énergie laser supérieure au seuil d'ablation du substrat de borosilicate ;
la formation sélective de canaux interconnectés sur au moins une partie de la surface du substrat de borosilicate à l'aide du faisceau de formation de motif, les canaux interconnectés ayant une seconde caractéristique de mouillage, la première caractéristique de mouillage et la seconde caractéristique de mouillage étant différentes ;
l'application sélective d'au moins un revêtement de substrat à revêtement de fluorocarbure hydrophobe sur le substrat de borosilicate et les canaux imbriqués ;et
l'ablation sélective d'au moins une partie du substrat de revêtement de fluorocarbure hydrophobe d'une partie du substrat de borosilicate pour faire apparaître les canaux imbriqués.
